# EUROPEAN PATENT SPECIFICATION

(11) **EP 4 424 294 B1**
(45) Date of publication and mention of the grant of the patent: **18.06.2025**
(21) Application number: 23159124.9
(22) Date of filing: 28.02.2023
(51) Int. Cl.: A61K 6/16, A61K 6/17, A61K 6/833, A61K 6/887, C03C 10/00, C03C 4/00

(54) **AN ESTHETIC, ROBUST, HYBRID AND BIOMIMETIC DENTAL COMPOSITION**
ÄSTHETISCHE, ROBUSTE, HYBRIDE UND BIOMIMETISCHE DENTALZUSAMMENSETZUNG
COMPOSITION DENTAIRE ESTHÉTIQUE, ROBUSTE, HYBRIDE ET BIOMIMÉTIQUE

(43) Date of publication of application: 04.09.2024
(73) Proprietor: DENTSPLY SIRONA Inc., York, PA 17401 (US); DeguDent GmbH, 63457 Hanau (DE)
(72) Inventor: VOLLMANN, Markus, 63403 Hanau (DE)
(74) Representative: Venner Shipley LLP

(56) References cited:
- EP-A1- 3 772 492
- US-A1- 2011 257 000
- US-B2- 7 316 740

## Description

### BACKGROUND OF THE INVENTION

The present invention relates to an esthetic, robust, hybrid, biomimetic and fast grindable and printable dental composition.

**Restoration** in the sense of the present invention is a dental restoration.

**Dental restoration** in the sense of the present invention is a crown (including inlay, onlay and partial crown), a bridge, an implant or another kind of a dental restoration.

**Restoration material** in the sense of the present invention is a dental restoration material.

**Platelets** in the sense of the present invention are particles made of an inorganic material, having a thickness of 0.2µm-10 µm.

The prior art dental compositions often need to be grinded, milled and polished for a long time or need a temperature treatment. Zirconia needs to be sintered at the temperature of 1500°C - 1600°C. Glass ceramics needs to be crystallized at the temperature of around 800°C. In addition to this, the prior art dental compositions often suffer from short life time in vivo and/or are esthetically challenged.

The Enamic material of the VITA company suffers from esthetic disadvantages, such as high opacity and staining after a short period in vivo. It is mainly due to the microstructure, which is an interpenetrating network of inorganic skeleton framework and an infiltrated organic polymer. Other prior arts CAD/CAM blocks have 'free floating' inorganic parts which have a high complexity of composition to adapt the refractive index to the organic material; the inorganic part composition includes a mixture of pure silica particles with the special glasses, for radio opacity, for example.

EP 3 772 492 A1 and US 2011/257000 A1 disclose dental compositions comprising an inorganic material and an organic material.

It is an object of the present invention to overcome the above shortcomings.

### DESCRIPTION OF THE INVENTION

PMMA in the sense of the present invention is Poly(methyl methacrylate).

Poly-UDMA in the sense of the present invention is Poly(urethane- dimethacrylate).

It is an object of the present invention to provide an esthetic, robust, hybrid and biomimetic dental composition, wherein the dental composition has long life time in vivo, it is fast grindable/millable without thermal post treatment. A dental restoration produced from the dental composition of the present invention requires brief polishing or applying organic based glaze only. Work-flow time for grinding, milling and polishing is less than 8 minutes, e.g. 4-5 minutes of grinding/milling and 2-3 minutes of manual and/or automatized polishing.

It is an object of the present invention to provide an esthetic, robust and hybrid dental composition for production of a dental restoration, wherein the structure and composition of the dental composition is similar to the natural tooth structure and composition, e.g. the dental composition is biomimetic. The natural tooth comprises hydroxyl-apatite crystal needles (an inorganic material) and collagen fiber (an organic material).

It is an object of the present invention to provide a dental composition as defined in claim 1 comprising an inorganic material and an organic material, wherein the inorganic material is in the form of platelets.

.In the preferred embodiment of the present invention, the inorganic material is a single composition high strength ceramic material present in the form of crystallized glass ceramic.

In the preferred embodiment of the present invention, the dental composition comprises 75 -95 weight % of the crystallized glass ceramic.

In the preferred embodiment of the present invention, the organic material fills the voids between the platelets.

In another embodiment of the present invention, the dental composition comprises another part of the single composition high strength ceramic material in the form of ceramic grains.

In the preferred embodiment of the present invention, the ceramic grains fill the voids between the platelets.

In the present invention, the platelets are of the thickness of 0.2 µm to10 µm, more preferably of 0.3 µm to 7 µm, more preferably of 0.4 µm to 5 µm and even more preferably of 0.5 µm to 3µm. The platelets have the aspect ratio of from 1:1 to 1:20.

In the preferred embodiment of the present invention, the organic material is a macromolecular material. The macromolecular material is preferably selected from the group comprising poly(methacrylates), including PMMA and Poly-UDMA. In another preferred embodiment of the present invention, the organic material is selected from the group comprising Ormocer.

The refractive index of the organic material is fitting with the refractive index of the inorganic material, which is very important for an optimal translucency of the dental composition.

In the preferred embodiment of the present invention, the difference between the refractive index of the inorganic material and of the organic material is not more than 0.1.

The distance between the platelets is in the range of several µm.

In the present invention, the inorganic material has the composition as defined in the Table No. 1.

**Table 1**

| Oxide | From (weight %) | To (weight %) |
|---|---|---|
| SiO₂ | 58,3 | 60,20 |
| P₂O₅ | 5,4 | 5,60 |
| Al₂O₃ | 2,5 | 2,50 |
| Li₂O | 14,4 | 14,70 |
| K₂O | 1,2 | 1,20 |
| ZrO₂ | 9,8 | 11,00 |
| CeO₂ | 0,5 | 1,90 |
| Tb₄O₇ | 1 | 1,70 |
| V₂O₅ | 0 | 0,8 |
| Er₂O₃ | 0,1 | 0,9 |
| Y₂O₃ | 0,4 | 0,4 |
| MnO₂ | 0 | 0,1 |
| Nd₂O₃ | 0 | 0,3 |
| Fe₂O₃ | 0 | 0,2 |
| B₂O₃ | 0 | 2,7 |
| Na₂O | 0,2 | 0,2 |

The dental composition of the present invention is for use for producing a dental restoration. The platelets will be crystallized to an amount of more than 60 wt% of lithium disilicate (LS2) crystals as a major crystal component combined with lithium aluminium silicates (LAS) as a minor crystal part (5-25% of all crystals).

The crystallized glass ceramic has biaxial strength (according ISO 6872) of more than 600 MPa due to the zirconia amount diluted in the amorphous phase and the mixture of the lithium disilicate ceramic with the LAS crystals. The platelet shape of the high strength glass ceramic results in a high possible filling grade starting which is still 3D printable by an extrusion process with 75-95% vol% of crystallized glass ceramic, resulting in a significant higher strength of the hybrid dental material (wherein crystallized glass ceramic prevails in the dental composition and the organic material content is relatively minor), which exceeds the biaxial strength by at least 50% of composites available in the market as CAD machinable blocks, which have biaxial strength of around 120-270 MPa. The dental hybrid material comes closer to the composition of a real tooth.

The hybrid dental material is preferable used for CAD/CAM machinable block material and is 3D printable (due to the platelet shape of the crystallized glass-ceramic). Therefore, every internal free-form for the CAD block can be used. Due to the composition of the crystallized glass ceramic (see table above) one is able to use only this (one) kind of the crystallized glass ceramic. Other composite materials are consisting of several different glass types, merely silica (SiO2) combined with special glasses for better radio opacity.

Due to content of zirconia the radio opacity is included in the high strength glass ceramic and therefore, only one type of glass is needed.

**Due to the adaptation of a relatively smaller part of the organic material** in the optical refractive index within all wave-length of the visible light (400-800 nm), the hybrid dental material can be adjusted in translucency from transparent to nearly opaque, matching the translucency gradient of natural tooth.

The hybrid dental material of the present invention as a block material has an excellent machinability due to the small shape of the crystallized glass ceramic platelets despite the high volume percentage of this crystallized glass ceramic.

The marginal edges of the dental restoration are very sharp in comparison to pure brittle dental ceramics as pure lithium silicate CAD blocks or zirconia. Due to the high strength directly after the machining no additional thermal treatment is needed which results in a faster chair-side workflow time for the patient. By omitting the firing cycle the crown or another kind of dental restoration can be designed, milled and pace within 20 minutes when the tooth has been prepared by the dentist and an optical scan of the prepared tooth has been obtained (within 1-2 minutes).

By producing the CAD block out of this hybrid dental material, one can use an extrusion printer even with a nozzle diameter down to 50 µm due to the platelet shape of the high strength ceramic material.

The shear force during the transition of the hybrid dental material through the nozzle results in a significant lowering of the viscosity while after transition it immediately relaxes to the higher viscosity (thixotropy). The fine diameter of the nozzle can be used to mimic the natural gradient of shade translucency of the natural tooth.

The dental restoration is a crown (including an inlay, an onlay and a partial crown), a bridge, an implant or another kind of a dental restoration.

In another embodiment of the present invention, the dental restoration comprises additionally an outer organic based glaze.

In the preferred embodiment of the present invention, the outer organic based glaze comprises PMMA or Poly-UDMA.

The outer organic based glaze reinforces the surface roughness of the dental restoration in order to reduce abrasion of the surface by contacts with an antagonist tooth or food.

The translucency of the dental composition can be adapted from ,window pane' translucency to semi-opacity, to adapt different translucency zones in the final dental restoration, which can therefore have a translucency gradient. When it comes to 3D-printing, different zones can be printed directly in a free shape.

## Claims

1. A dental composition comprising an inorganic material and an organic material, wherein the inorganic material is in the form of platelets,
the platelets are of the thickness of 0.2µm to 10µm and have the aspect ratio of from 1:1 to 1:20,
wherein the inorganic material has the following composition:
| Oxide | From (weight %) | To (weight %) |
|---|---|---|
| SiO₂ | 58,3 | 60,20 |
| P₂O₅ | 5,4 | 5,60 |
| Al₂O₃ | 2,5 | 2,50 |
| Li₂O | 14,4 | 14,70 |
| K₂O | 1,2 | 1,20 |
| ZrO₂ | 9,8 | 11,00 |
| CeO₂ | 0,5 | 1,90 |
| Tb₄O₇ | 1 | 1,70 |
| V₂O₅ | 0 | 0,8 |
| Er₂O₃ | 0,1 | 0,9 |
| Y₂O₃ | 0,4 | 0,4 |
| MnO₂ | 0 | 0,1 |
| Nd₂O₃ | 0 | 0,3 |
| Fe₂O₃ | 0 | 0,2 |
| B₂O₃ | 0 | 2,7 |
| Na₂O | 0,2 | 0,2 |
and wherein the inorganic material has biaxial strength of more than 600 MPa, measured according to ISO 6872.

2. The dental composition according to claim 1, wherein the organic material fills the voids between the platelets.

3. The dental composition according to claims 1-2, wherein the organic material is selected from the group comprising poly(methacrylates).

4. The dental composition according to any of the preceding claims, wherein the difference between the refractive index of the inorganic material and the organic material is not more than 0.1.

5. Use of the dental composition according to any of the preceding claims for producing a dental restoration.

6. The dental restoration of claim 5, wherein the dental restoration is a crown, a bridge, an implant or another kind of a dental restoration.

7. The dental restoration of claims 5-6, comprising additionally an outer organic based glaze.

## Patentansprüche

1. Dentalzusammensetzung, umfassend ein anorganisches Material und ein organisches Material, wobei das anorganische Material in Form von Plättchen vorliegt, wobei die Plättchen von einer Dicke von 0,2 µm bis 10 µm sind und ein Seitenverhältnis von 1:1 bis 1:20 aufweisen, wobei das anorganische Material die folgende Zusammensetzung aufweist:
| Oxid | Von (Gewicht-%) | Bis (Gewicht-%) |
|---|---|---|
| SiO₂ | 58,3 | 60,20 |
| P₂O₅ | 5,4 | 5,60 |
| Al₂O₃ | 2,5 | 2,50 |
| Li₂O | 14,4 | 14,70 |
| K₂O | 1,2 | 1,20 |
| ZrO₂ | 9,8 | 11,00 |
| CeO₂ | 0,5 | 1,90 |
| Tb₄O₇ | 1 | 1,70 |
| V₂O₅ | 0 | 0,8 |
| Er₂O₃ | 0,1 | 0,9 |
| Y₂O₃ | 0,4 | 0,4 |
| MnO₂ | 0 | 0,1 |
| Nd₂O₃ | 0 | 0,3 |
| Fe₂O₃ | 0 | 0,2 |
| B₂O₃ | 0 | 2,7 |
| Na₂O | 0,2 | 0,2 |
und wobei das anorganische Material eine biaxiale Festigkeit von mehr als 600 MPa aufweist, gemessen nach ISO 6872.

2. Dentalzusammensetzung nach Anspruch 1, wobei das organische Material die Hohlräume zwischen den Plättchen ausfüllt.

3. Dentalzusammensetzung nach Ansprüchen 1-2, wobei das organische Material aus der Gruppe ausgewählt ist, die Poly(methacrylate) umfasst.

4. Dentalzusammensetzung nach einem der vorhergehenden Ansprüche, wobei die Differenz zwischen dem Brechungsindex des anorganischen Materials und des organischen Materials nicht mehr als 0,1 beträgt.

5. Verwendung der Dentalkomposition nach einem der vorhergehenden Ansprüche zum Herstellen einer Dentalrestauration.

6. Dentalrestauration nach Anspruch 5, wobei die Dentalrestauration eine Krone, eine Brücke, ein Implantat oder eine andere Art einer Dentalrestauration ist.

7. Zahnrestauration nach Ansprüchen 5-6, zusätzlich umfassend eine äußere organische Glasur.

## Revendications

1. Composition dentaire comprenant un matériau inorganique et un matériau organique,
ledit matériau inorganique se présentant sous la forme de plaquettes
lesdites plaquettes présentant une épaisseur de 0,2 µm à 10 µm et un rapport de forme allant de 1:1 à 1:20,
ledit matériau inorganique présentant la composition suivante :
| Oxyde | De (% en poids) | À (% en poids) |
|---|---|---|
| SiO₂ | 58,3 | 60,20 |
| P₂O₅ | 5,4 | 5,60 |
| Al₂O₃ | 2,5 | 2,50 |
| Li₂O | 14,4 | 14,70 |
| K₂O | 1,2 | 1,20 |
| ZrO₂ | 9,8 | 11,00 |
| CeO₂ | 0,5 | 1,90 |
| Tb₄O₇ | 1 | 1,70 |
| V₂O₅ | 0 | 0,8 |
| Er₂O₃ | 0,1 | 0,9 |
| Y₂O₃ | 0,4 | 0,4 |
| MnO₂ | 0 | 0,1 |
| Nd₂O₃ | 0 | 0,3 |
| Fe₂O₃ | 0 | 0,2 |
| B₂O₃ | 0 | 2,7 |
| Na₂O | 0,2 | 0,2 |
et ledit matériau inorganique présentant une résistance biaxiale supérieure à 600 MPa, mesurée selon la norme ISO 6872.

2. Composition dentaire selon la revendication 1, ladite matière organique remplissant les vides entre les plaquettes.

3. Composition dentaire selon les revendications 1 à 2, ledit matériau organique étant choisi dans le groupe comprenant les poly(méthacrylates).

4. Composition dentaire selon l'une quelconque des revendications précédentes, la différence entre l'indice de réfraction du matériau inorganique et du matériau organique n'étant pas supérieure à 0,1.

5. Utilisation de la composition dentaire selon l'une quelconque des revendications précédentes pour produire une restauration dentaire.

6. Restauration dentaire selon la revendication 5, ladite restauration dentaire étant une couronne, un bridge, un implant ou un autre type de restauration dentaire.

7. Restauration dentaire selon les revendications 5 à 6, comprenant en outre une glaçure externe à base organique.
